# EUROPEAN PATENT SPECIFICATION

(11) **EP 1 020 477 B1**
(45) Date of publication and mention of the grant of the patent: **30.06.2004**
(21) Application number: 96934330.0
(22) Date of filing: 11.10.1996
(51) Int. Cl.: C07J 71/00, A01N 45/00

(54) **POLYHYDROXYSPIROSTANNONES AS PLANT GROWTH REGULATORS**
POLYHYDROXYSPIROSTANNONE ALS PFLANZENWACHSTUMSREGULATOREN
POLYHYROXYSPIROSTANONES EN TANT QUE REGULATEURS DE LA CROISSANCE VEGETALE

(30) Priority: 12.10.1995 CU 9295
(43) Date of publication of application: 19.07.2000
(73) Proprietor: Coll Manchado, Francisco, Ciudad Habana 11400 (CU); Jomarron Rodiles, Isabel Maria, Ciudad Habana 11700 (CU); Robaina Rodriguez, Caridad Marcelina, Ciudad Habana 10400 (CU); Alonso Becerra, Esther Maria, Ciudad Habana 10400 (CU); Cabrera Pedroso, Maria Teresa, Ciudad Habana 11300 (CU)
(72) Inventor: Coll Manchado, Francisco, Ciudad Habana 11400 (CU); Jomarron Rodiles, Isabel Maria, Ciudad Habana 11700 (CU); Robaina Rodriguez, Caridad Marcelina, Ciudad Habana 10400 (CU); Alonso Becerra, Esther Maria, Ciudad Habana 10400 (CU); Cabrera Pedroso, Maria Teresa, Ciudad Habana 11300 (CU)
(74) Representative: Smulders, Theodorus A.H.J., Ir.
(86) International application number: PCT/CU1996/000002
(87) International publication number: WO 1997/013780

## Description

This invention is related to the chemical-biological field and in particular to a procedure to obtain new spirostan analogs of brassinosteroids based on steroidal sapogenins, through partial synthesis. These spirostan analogs have a regulating effect on plant growth.

Brassinosteroids are steroidal compounds, with a regulating activity on plant-growth and which are found in very small quantities in plants. For this reason extracting them from plants is economically prohibitive. Some of the natural compounds have been obtained sinthetically but with very low yields, thus the current tendency is to synthetisize analogs of these compounds.

The majority of them possess (among other typical structural requirements), a 2α,3α- diol system in the A ring and a union trans of the A and B rings, of the steroidal system. Many scientists attribute a relevant importance to this peculiarity because of the biological activity that they are able to carry out.

For that reason, some of the analogs of synthesized brassinosteroids present substituents in positions 2 and 3 of the A ring with alfha stereochemical, which exhibit activity as regulators of plant growth, (Kohout, L., Strnad, M., *Collect. Czech. Chem. Commun.* **57**, 1731, **1992**; Cerny,V., et al *Collect. Czech. Chem. Commun*. **51**, 687, **1986**.)

Also, the first similar spirostanics of brassinosteroids synthesized starting from steroidals sapogenin, possess α stereochemistry /orientation of the sustituents groups in the A ring in the positions 2,3 (Marquardt, V. et al, *German Patent CO7J 63*/*00 273638A*, **1989**), but the synthesis of them involves the utilization of a very toxic reagent, osmium tetroxide.

It is also interesting to study how variations in the positions of these hydroxylic groups in the A ring influence the biological activity that these compounds exert. The synthesis of compounds shown in this invention possess oxigenated function in the positions 3,5 with different stereochemistry, for example : the (25R) 3β-acetoxy-5-hydroxy-5α-spirostan-6-one **2a**, (25R) 3β,5-dihydroxy-5α-spirostan-6-one **2b** and (25R) 3β,5-dihydroxy-5β-spirostan-6-one **2c**. These structural characteristics is unusual for this type of compounds with plant growth promoting activity.

In 1975, the compound (25R) 3β,5-dihydroxy-5α-spirostan-6-one **2b** was synthesized by Novosel'skaya, I.L. et al (*Chemistry of Natural Compounds,* Vol **11**, No 2, 268-269, **1975**) as intermediate for the purpose to obtain the ecdysones analogs from steroid sapogenins. The diosgenin was converted into the triol (25R) 5α-spirostane-3β,5,6β-triol through the method reported by Romo, J. et al (*J. Org. Chem*., **19**, 1509, **1954**) with hydrogen peroxide/formic acid. On oxidation of the triol with N-bromosuccinimide in dioxane solution for seven days formed the compound **2b** (Nawa, H. et al, *Chem*. *Pharm. Bull. Jpn.* 11, 139, **1963**). The acetylation of the compound **2b** gave the compound **2a.**

By the same way, in 1982, Dawidar, A.M. et al *(Zeitschrift für Naturforschung*, *part B : Anorganische Chemie, Organische Chemie,* vol **37B**, No 7, 892-895, **1982**) reported the synthesis of the compound **2b** with in order to prepare 6-keto diosgenin derivatives which could be converted to the intermediate 6-methyl pregnane compounds.

The reaction of diosgenin with hydrogen peroxide/formic acid has the inconvenient to produce 5α-pregnane-3β,5,6β,16β,20α-pentol (POL) as a by product. By the other hands, oxidation process with NBS required several days.

In 1984 Schwantz, R. et al, (*Revue Roumanie of Chimie*, **29**, 9-10, 755-759, **1984**) published the synthesis of the (25R) 3β-acetoxy-5-hydroxy-5α-spirostan-6-one (2a) as an intermediary compound for the synthesis of 3β-acetoxy-B-nor-androst-5-en-3β-ol-17-one and 3β-acetoxy-B-nor-pregn-5-en-3β-ol-20-one, by means of acetilation reactions with acetic anhydride and pyridine, to obtain the acetate of diosgenin. Then an epoxidation of the double bond with sodium acetate, chloroform and peracetic acid at 14.5%, is performed to obtain the corresponding epoxide and subsequently it is oxidized utilizing acetone and chromic anhydride dissolved in water.

In the present invention, with the purpose of obtaining the compound **2b** in mass quantities, the previously described methods were repeated, until (25R)3β-acetoxy-5-hydroxy-5α-spirostan-6-one **2a**, noting that in the epoxide oxidation with the above mentioned conditions, the reaction takes place with difficulty and low yields to produce the corresponding ketol.

Also this process requires the acetylation of diosgenin with acetic anhydride and pyridine at room temperature for 24 hours, which, in addition to the time required involves the utilization of a pyridine reagent which is a toxic and relative expensive reagent.

Bearing this in mind, it was decided to develop a procedure for the synthesis of spirostanones with oxygenating functions at 3,5 positions of the A ring with a different stereochemistry employing a feasible, continuous and higher yield procedures in their preparation. The method used to obtain new spirobrassinosteroids with the general formula is described below:

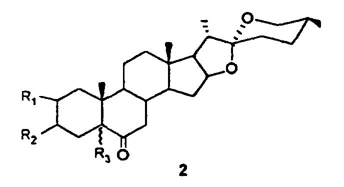

| Compounds | R₁ | R₂ | R₃ |
|---|---|---|---|
| 2a | H | CH₃COO β | HO α |
| 2b | H | HO β | HO α |
| 2c | H | HO β | HO β |

The compounds with these general formulas present a regulating activity on plant growth.

Diosgenin was direct acetilated by heating with (1-3) part in volume of acetic anhydride, the wet diosgenin acetate collected by filtration was suspended in (20-40) part in volume of acetone and treated with acetic anhydride/H₂O₂ at 35 % at room temperature, followed by another oxidation with (1-3) parts in volume of the Jones reagent.

These process can be realized in another solvents such as methylethylketone, isopropylmethylketone, isobutylmethylketone, dichloromethane, 1,2-dichloroethane, etc.

The saponification of **2a** utilizing (10-20) part in volume potassium hydroxide dissolved in 5% ethanol produces compound 2b. The C-5 isomerization of the compound **2b** produces **2c**.

The synthetic scheme followed to obtain the products was:

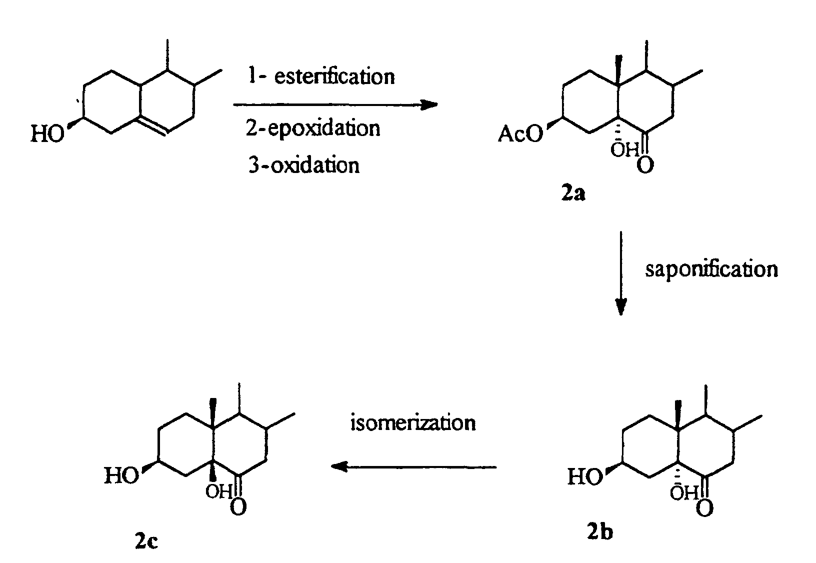

This invention made possible to obtain three new spirobrassinosteroids with oxygenating functions in 3,5 positions of the A ring with a different stereochemistry, that demonstrate activity as controllers of plant growth.

The synthetic process utilizes common chemical reagents with low toxicity.

The synthesized compounds facilitate the increased growth and development of the plants, as well as their agricultural yields. They are additionally use as an additive in the post-harvest conservation of the flowers.

### Example of the process.

To a suspension containing 2g of diosgenin in 6 mL of anhydride acetic was heated under reflux for 1.5 h.. When the reaction was concluded, was cooled and the diosgenin acetate was filtered. The wet acetate was suspended in 10 mL of acetone and treated under mechanical stirring with 2mL of anhydride acetic, 0.6 g of sodium acetate, 1.4 mL of H₂O₂ were added dropwise at 0-10 °C. When the reaction was concluded, the mixture washed with a saturated solution of sodium chloride and sodium hydrogen carbonate. The organic phase was heat under refluxing, 1.5 mL of Jones reagent were added dropwise. When the reaction was concluded, the usual work-up afforded the product **2a**. Yield: 2.2 g (95%).

The compounds referred in this invention can be utilized as the active principles of a formulation that presents properties as vegetal growth regulator and as an additive for the post-harvest conservation of flowers.

As regulator of plant growth the compounds in this invention can be prepared in liquid form or in a water solution, mixing the active ingredient with one or various types of liquid agents including organic solvents such as ethanol (500-1000 mL) and dimethylformamide (20-60g), among others. If can be applied as well as a solid, or a liquid formulation, etc, mixed with fertilizers, pesticides, herbicides, etc.

When preparing the formula an active surface agent such as tween-20 (0.05-0.1 %) can be added to improve its dispersability, when preparing a water solution for the concentrated liquid formula for its application to the plant. This formulation results stable to temperatures less than 35°C for periods until one year.

The compounds referred in this invention can be applied in concentrations of 10⁻⁴ to 10 ppm and in a dose within the range of 0.1 to 1000 mg/ha depending on the vegetative state of the plant and the edafoclimatics conditions, among other factors.

The property as plant growth regulator of the synthesized products was determinated by means of bioassays :
1. Hypocotyl elongation and expansion of the radish colyledons
2. Retention of the degradation of the photosynthetic pigments in wheat leaves.

In the first bioassay, solutions with a concentration of the active principle between 0.01-10 [ppm] were employed, to obtain an increase in the length of the hypocotyl between 1.1 ± 0.22 cm, as well as an increment in the weight of the cotyledons. Moreover, the bioactivity of the compounds was corroborated by means of the same field test up to 1 Ha with various cultivars, for example:

| CULTIVAR | CONCENTRATION | YIELD INCREASE (AVERAGE %) |
|---|---|---|
| Onion | 0.1-1 ppm | 21-40 % |
| Pepper | 0.5-1 ppm | 8-50 % |
| Garlic | 0.1-0.5 ppm | 2-15 % |
| Potato | 0.1-10 ppm | 18-25 % |
| Corn | 0.1-0.5 ppm | 10-47 % |
| Soy bean | 0.1-1 ppm | 9-34 % |
| Sorghum | 0.1-1 ppm | Aproxim. 15 % |
| Rice | 0.1-1 ppm | 12 % |
| Tomato | 0.1-1 ppm | 6-11% |

## Claims

1. Spirostanones with oxygenating functions in the A ring, as regulator of growth and their preparation procedure, **characterized by** general formula (2): 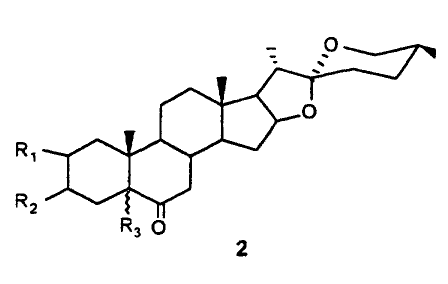 where:
| Compounds | R₁ | R₂ | R₃ |
|---|---|---|---|
| 2a | H | CH₃COO β | HO α |
| 2b | H | HO β | HO α |
| 2c | H | HO β | HO β |

2. The procedure for the synthesis of spirobrassinosteroids derivatives according to claim No. 1 is characterized because such compounds are obtained by means of steroidal sapogenin through the following chemistry reactions::
a- The compound **2a** is obtained through direct oxidation of diosgenin acetate utilizing between (20-40), (1-3) and (0.5-1) parts in volume of acetone, acetic anhydryde and hydrogen peroxide at 35% respectively and sodium acetate in molar proportion of (1-2) followed by a further oxidation with (1-3) parts in volume of Jones reagent.
b- The saponification of **2a** utilizing (10-20) part in volume of potassium hydroxyde disolution in 5% ethanol yield the compounds **2b**.
c- The C-5 isomerization of the compound **2b** produces **2c**.

3. Spirostanones obtained according to claims 1 and 2 are characterized because their are used as regulator of vegetal growth.

4. Spirostanics analogues of brassinosteroids according to claims from 1 to 3 are characterized because they are utilized as regulators of growth plant and as flowers preservative.

5. Formulation that contains spirostanones or spirostanics analogues of brassinosteroids of claims (1 and 4), is **characterized by** being a liquid form that contains between 10⁻⁴- 100 ppm of the active principle and can be applied alone or mixed with herbicides, pesticides, fertilizers, etc, that are soluble in water.

6. Use of spirostanones or spirostanics analogues of brassinosteroids of former claims as regulators of growth plant and like flowers preservative.

## Patentansprüche

1. Spirostanone mit oxidierenden Funktionen im A-Ring als Wachstumsregulatoren und Verfahren zu ihrer Herstellung, **gekennzeichnet durch** die allgemeine Formel (2): 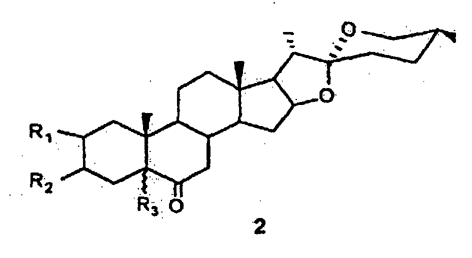 worin:
| Verbindungen | R₁ | R₂ | R₃ |
|---|---|---|---|
| 2a | H | CH₃COO β | HO α |
| 2b | H | HO β | HO α |
| 2c | H | HO β | HO β |

2. Verfahren zur Synthese von Spirobrassinosteroid-Derivaten nach Anspruch 1 ,
**dadurch gekennzeichnet, daß** diese Verbindungen mittels eines Steroid-Sapogenins durch folgende chemische Reaktionen erhalten werden:
a) Die Verbindung 2a wird durch direktes Oxidieren von Diosgeninacetat unter Verwendung von (20 - 40), (1 - 3) und (0,5 - 1) Volumenteilen Aceton, Essigsäureanhydrid bzw. Wasserstoffperoxid mit 35 % und Natriumacetat in einem Molverhältnis von (1 - 2), gefolgt vom weiteren Oxidieren mit (1 - 3) Volumenteilen Jones-Reagenz erhalten;
b) die Verseifung von 2a unter Verwendung von (10 - 20) Volumenteilen einer Kaliumhydroxidlösung in 5 % Ethanol ergibt die Verbindungen 2b;
c) die C-5-Isomerisierung der Verbindung 2b erzeugt 2c.

3. Spirostanone, die gemäß den Ansprüchen 1 und 2 erhalten wurden, **dadurch gekennzeichnet, daß** sie als Pflanzenwachstumsregulatoren verwendet werden.

4. Dem Spirostan analoge Verbindungen von Brassinosteroiden gemäß den Ansprüchen 1 bis 3, **dadurch gekennzeichnet, daß** sie als Pflanzenwachstumsregulatoren und als Konservierungsmittel für Blumen verwendet werden.

5. Formulierung, die Spirostanone oder dem Spirostan analoge Verbindungen von Brassinosteroiden gemäß den Ansprüchen 1 und 4 enthält, **dadurch gekennzeichnet, daß** sie in flüssiger Form vorliegt, die 10⁻⁴ - 100 ppm des aktiven Grundbestandteils enthält und allein oder in einem Gemisch mit Herbiziden, Pestiziden, Düngemitteln usw., die wasserlöslich sind, angewandt werden kann.

6. Verwendung von Spirostanonen oder dem Spirostan analogen Verbindungen von Brassinosteroiden gemäß den vorstehenden Ansprüchen als Pflanzenwachstumsregulatoren und ähnliche Konservierungsmittel für Blumen.

## Revendications

1. Spirostanones ayant des fonctions d'oxygénation dans le cycle A, en tant que régulateur de croissance, et leur procédé de préparation, **caractérisés par** la formule générale (2) : 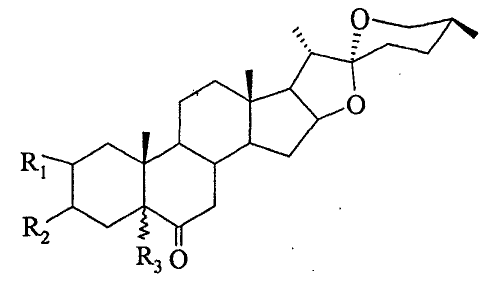 où
| Composés | R₁ | R₂ | R₃ |
|---|---|---|---|
| 2a | H | CH₃COO β | HO α |
| 2b | H | HO β | HO α |
| 2c | H | HO β | HO β |

2. Procédé de synthèse de dérivés de spirobrassinostéroïdes selon la revendication 1, **caractérisé en ce que** de tels composés sont obtenus au moyen de sapogénine stéroïde par l'intermédiaire des réactions chimiques suivantes :
a- Le composé **2a** est obtenu par oxydation directe d'acétate de diosgénine en utilisant respectivement de (20 à 40), (1 à 3) et (0,5 à 1) parties en volume d'acétone, d'anhydride acétique et de peroxyde d'hydrogène à 35%, et de l'acétate de sodium en proportion molaire de (1 à 2), suivie par une nouvelle oxydation avec (1 à 3) parties en volume de réactif de Jones.
b- La saponification de **2a** en utilisant (10 à 20) parties en volume de solution d'hydroxyde de potassium à 5% dans l'éthanol aboutit aux composés 2b.
c- L'isomérisation en C-5 du composé **2b** fournit **2c**.

3. Spirostanones obtenus selon les revendications 1 et 2, **caractérisés en ce qu'**ils sont utilisés en tant que régulateur de la croissance végétale.

4. Analogues spirostaniques de brassinostéroïdes selon les revendications 1 à 3, **caractérisés en ce qu'**ils sont utilisés en tant que régulateurs de la croissance des plantes et en tant que conservateurs pour fleurs.

5. Formulation contenant des spirostanones ou des analogues spirostaniques de brassinostéroïdes selon les revendications 1 et 4, **caractérisée en ce qu'**elle est sous forme liquide contenant entre 10⁻⁴ et 100 ppm de principe actif et peut être appliquée seule ou mélangée à des herbicides, des pesticides, des engrais, etc., qui sont solubles dans l'eau.

6. Utilisation de spirostanones ou d'analogues spirostaniques de brassinostéroïdes selon les revendications précédentes, en tant que régulateurs de la croissance des plantes et conservateurs pour fleurs analogues.
